Europäisches Patentamt

**European Patent Office**

Office européen des brevets

(11) Numéro de publication: **0 277 930**
**A1**

## (12) DEMANDE DE BREVET EUROPEEN

(21) Numéro de dépôt: 88870002.8

(22) Date de dépôt: 13.01.88

(51) Int. Cl.⁴: **A 61 K 39/00**
A 61 K 39/002, A 61 K 39/02,
A 61 K 39/12, G 01 N 33/569,
C 07 K 17/00

(30) Priorité: 15.01.87 LU 86736

(43) Date de publication de la demande:
10.08.88 Bulletin 88/32

(84) Etats contractants désignés: **ES GR**

(71) Demandeur: **UNIVERSITE LIBRE DE BRUXELLES**
**50, avenue F.D. Roosevelt**
**B-1050 Bruxelles (BE)**

(72) Inventeur: **Legros, Franz**
**313 chaussée de Boondael**
**B-1050 Bruxelles (BE)**

**Ruysschaert, Jean-Marie**
**7, avenue du Perce-Neige**
**B-1640 Rhose-Saint-Genese (BE)**

(74) Mandataire: **Van Malderen, Michel et al**
**p.a. Freylinger & Associés 22 avenue J.S. Bach (bte 43)**
**B-1080 Bruxelles (BE)**

(54) Liposomes sensibilés à des molécules antigéniques de parasites intracellulaires, procédé pour leur préparation, et leurs applications comme moyens de diagnostic et comme agents d'immunisation.

(57) Agents convenant comme moyens de diagnostic et comme agents d'immunisation (vaccins) de parasites intracellulaires, lesdits agents étant constitués par des liposomes sensibilisés à des molécules antigéniques spécifiques de parasites intracellulaires, par ex. la tuberculine, la Old tuberculin, la lépromine ou la HTLV3. Le procédé de production de ces liposomes sensibilisées peut consister en une encapsulation de la molécule antigénique ou en la mise en contact du liposome préformé avec ladite molécule.

EP 0 277 930 A1

**Description**

LIPOSOMES SENSIBILISÉS À DES MOLÉCULES ANTIGÉNIQUES DE PARASITES INTRACELLULAIRES, PROCÉDÉ POUR LEUR PRÉPARATION ET LEURS APPLICATIONS COMME MOYENS DE DIAGNOSTIC ET COMME AGENTS D'IMMUNISATION

Objet de l'invention

La présente invention repose sur la sensibilisation des liposomes à des molécules antigéniques de parasites intracellulaires et ouvre de ce fait des applications nouvelles de tels liposomes sensibilisés, comme moyens de détection d'Ig (lytiques ou non lytiques) contre ces antigènes infectieux dans des sera ou autres liquides organiques (pleuraux, péritonéaux, de lavage bronchoalvéolaire) et comme agents de vaccination capables de susciter une immunité (cellulaire, mais aussi humorale) contre l'agent infectant.

À titre d'application particulière illustrée par la présente invention, on vise plus spécifiquement à fournir des moyens de diagnostic et d'immunisation pour la tuberculose mais l'invention n'est bien entendu pas limitée à cette application, ainsi qu'il apparaîtra ci-après.

Résumé de l'état de la technique

De nombreux travaux ont été consacrés aux liposomes pour diverses applications pharmacologiques, notamment comme vecteurs de substances à activité médicamenteuse.

Des molécules antigéniques, naturelles ou hapténiques, ont également été exposées à la surface de liposomes. Ces liposomes dits sensibilisés ont été utilisés soit pour la détection d'immunoglobulines dans les sera d'animaux immunisés soit comme adjuvants d'immunité susceptibles d'augmenter l'immunisation humorale, mais aussi cellulaire.

Pour ce qui concerne la tuberculose, il existe des extraits protéiques purifiés de différentes Mycobacteria et plus particulièrement de Mycobacterium tuberculosis var. hominis (H37Rv) = PPD H37Rv = Tuberculine H37Rv Les tuberculines sont capables de révéler une immunité cellulaire préexistante chez un homme ou un animal vacciné ou infecté (Intradermoréaction ou réaction d'hypersensibilité retardée), mais elle est incapable d'immuniser car elle n'est pas immunogène.

Éléments caractéristiques de l'invention

L'invention porte spécifiquement à titre d'agents nouveaux pouvant servir aux fins précitées (immunoessai et vaccination) sur des liposomes sensibilisés à des molécules antigéniques spécifiques de parasites intracellulaires.

Ces liposomes sont de préférence du type dit "multilamellaires" (MLV) avec ou sans cholestérol.

On accorde la préférence, selon l'invention, généralement à des liposomes multilamellaires avec cholestérol présentant des rapports Egg Pc/Ch compris entre 4/1 et 4/4, ce dernier rapport ou des valeurs proches de celui-ci étant tout particulièrement préférés.

L'invention porte également sur des procédés de production de liposomes sensibilisés.

Deux variantes opératoires s'offrent à cet effet:

Il est possible de procéder à une encapsulation directe de molécules antigéniques ou de mettre en contact le liposome préformé avec ces molécules.

Les deux types de liposomes sensibilisés sont capables de fixer des immunoglobulines dressées contre les molécules antigéniques.

On observe généralement dans le premier cas que plus d'immunoglobulines sont liées aux liposomes tandis que les protéines incorporées par exposition à des vésicules préformées présentent une affinité plus élevée pour les anticorps correspondants. Il en résulte que le type de sensibilisation des liposomes à un mélange d'immunoglobulines correspond à des quantités variables de molécules antigéniques choisies et exposées à la surface des liposomes et à des différences dans l'exposition et l'affinité de ces antigènes.

L'administration répétée, sous-cutanée ou intrapéritonéale, des deux types de liposomes suscite l'apparition d'une réaction d'hypersensibilité retardée, témoin d'une immunité de type cellulaire, chez le cobaye.

La liste des applications spécifiques relevant de l'invention figure ci-après dans le mémoire descriptif.

Description détaillée de mode opératoire en référence à l'application particulière du Mycobacterium tuberculosis

Méthode de sensibilisation des liposomes

1. Formation des liposomes
Concentration totale des lipides : 10 ou 20 mg/ml.

Rapports molaires 1) Egg Pc (ou Egg Pc = 4/Ch = 0)

2) Egg Pc/Ch 4 : 1

4 : 2

4 : 3

4 : 4

Formation des MLV dans NaCl 0,15 M (= Eau physiologique) stérile avec ou sans PPD.
Deux méthodes de sensibilisation sont réalisées :

a. Encapsulation directe de la PPD dans les liposomes. Les liposomes sont formés dans une solution de PPD à 0,75 mg/ml de NaCl 150 mM.

La préparation est laissée au repos pendant 30 min. à température ambiante.

Puis, 5 rinçages dans NaCl 150 mM.

b. Mise en contact de liposomes préformés avec la PPD. Les MLV sont formés dans le NaCl 150 mM, laissés au repos pendant 30 min., centrifugés à 3500 g pendant 10 min., mis en contact pendant 30 min avec une solution de PPD à 0,75 mg/ml de NaCl 150 mM et rincés 5 fois dans le même solvant.

## 2. Radioimmunoessai utilisant des liposomes sensibilisés

Des liposomes de différentes compositions et sensibilisés selon les deux méthodes sont exposés à un antiserum de mouton dressé contre H37Rv, radioiodé et non marqué. La préparation et la radioiodation de cet antiserum, est décrite dans la demande de brevet de la Demanderesse déposée ce même jour pour : "Procédé de détection rapide d'infections à germes intracellulaires, en particulier de Mycobacterium tuberculosis ou de Mycobacterium leprae et trousse convenant à cet effet". Des courbes de compétition sont obtenues entre une quantité d'antisérum $^{125}$I et différentes dilutions de l'antisérum non marqué (de 1/10 à 1/10 000). La proportion de molécules radioiodées fixées aux liposomes-PPD et la constante de dissociation des molécules sont mesurées. La composition liposomiale qui fixe le plus grand nombre de molécules radioiodées avec la plus grande affinité semble être Egg Pc/Ch 4 : 4 avec encapsulation directe de la PPD.

## 3. Radioimmunoessais sur sera humains.

Des compétitions ont été effectuées entre l'antiserum de mouton radioiodé et différentes dilutions de sera humains (1/10 à 1/10 000). Aucune compétition n'est observée pour les sera de patients non-tuberculeux. Par contre, 6 sera de patients tuberculeux contenaient des Ig entrant en compétition avec l'antiserum-[125] I. La présence d'Ig dans les sera de ces patients a été confirmée par test ELISA. Indem pour l'absence d'Ig dans les sera des patients non-tuberculeux. Liposomes utilisés dans cette étude .:Egg Pc/Ch 4 : 3; encapsulation directe de la PPD ou exposition de la PPD à des liposomes préformés. Ce radioimmunoessai détecte des Ig dans les sera de patients tuberculeux, mais ne renseigne pas:sur la nature de ces Ig.

## 4. Mise au point d'un immunoessai avec marqueur fluorescent = Essai de Lyse Immune des Liposomes.

Un marqueur fluorescent, la calcéine, a été incorporé dans les liposomes Egg Pc/Ch (4 : 3) sensibilisés à la PPD par encapsulation directe. Tant qu'il est encapsulé, donc "quenché", ce marqueur ne fluoresce pas. Si le liposome est lysé, le marqueur est libéré dans le milieu d'incubation et une fluorescence proportionnelle à l'importance de la lyse liposomiale peut être mesurée.

La lyse des liposomes a lieu si des IgG lytiques sont venues se fixer aux antigènes exposés par les vésicules (ici, des antigènes extraits de la PPD) en présence de complément. Qualitativement, cet immunoessai est le seul actuellement à détecter des Ig lytiques.

Les faux positifs sont exclus puisque la lyse liposomiale n'a lieu que si les Ig se sont fixées aux antigènes exposés par les vésicules. En outre, si la raimmunoessai cité en 3) nécessite 20 mg de lipides par point de compétition, l'essai de lyse immune avec la calcéine ne nécessite que 0,1 mg pour la même mesure.

Des liposomes constitués d'Egg PC et Ch (4 : 3), renfermant de la calcéine et sensibilisés à la PPD selon les deux méthodes indiquées plus haut (encapsulation et mise en contact), ont présenté une lyse immune (libération de calcéine et apparition d'une fluorescence mesurable dans le milieu) en présence de serum de lapine anti-BCG (Dakopatts) ou de serum de mouton anti Mycobacterium tuberculosis et de complément provenant de serum de lapin. Cette réaction n'a pas été observée en présence d'un serum connu comme ne présentant pas d'anticorps antimycobactériens ni en l'absence de complément.

Ces mêmes liposomes sensibilisés à la PPD ont été mis en contact avec des sera humains de patients tuberculeux et non-tuberculeux intradermo-négatifs. Les lyses mesurées (L.S.) après 30 minutes ont été comparées à celles trouvées pour des liposomes non sensibilisés à la PPD ni à aucun autre antigène protéique (LNS). Pour chaque serum, la différence entre les pourcentages L.S. et LNS a été calculée (LILA). Les résultats obtenus snt repris dans le tableau suivant.

| Patients tuberculeux | | | | Patients non-tuberculeux | | |
|---|---|---|---|---|---|---|
| | LS | LNS | LILA | | LS | LNS | LILA |
| 1 | 23,7 | 12,4 | 11,1 | 11 | 11 | 6,8 | 4,2 |
| 2 | 31 | 7 | 24 | 12 | 3,8 | 3,7 | 0,1 |
| 3 | 31 | 13 | 18 | 13 | 12,4 | 5,2 | 7,2 |
| 4 | 20 | 9,5 | 10,5 | 14 | 11 | 3,6 | 7,4 |
| 5 | 14,7 | 10,6 | 4,1 | 15 | 7,7 | 2,6 | 5,1 |
| 6 | 39 | 14 | 25 | 16 | 3,8 | 1,3 | 2,5 |
| 7 | 57 | 15,6 | 41,4 | 17 | 3 | 2 | 1 |
| 8 | 32 | 8 | 24 | 18 | 4,6 | 2,9 | 1,5 |
| 9 | 25 | 12 | 13 | 19 | 6 | 1 | 5 |
| 10 | 31 | 15,8 | 15,3 | 20 | 3 | 1 | 2 |
| M | 30,4 | 11,8 | 18,7 | | 6,6 | 3 | 3,6 |
| ±SD | ±11 | ±2,8 | ±10 | | ±3,5 | ±1,8 | ±2,4 |

S     S          NS     NS

S

S

S

S = statistiquement significatif selon le test de student

NS = statistiquement non significatif selon le test de student

De ces résultats, il ressort que la lyse immune des liposomes porteurs de PPD est significativement plus élevée dans les sera de tuberculeux que dans les sera de non-tuberculeux. De même, la lyse des liposomes

4

non porteurs de PPD est plus grande dans les sera tuberculeux, bien que significativement inférieure à celle des liposomes PPD. Le serum des patients tuberculeux contient donc des anticorps dirigés contre les composants de la PPD, mais aussi des agents susceptibles de provoquer une lyse de liposomes mais PPD plus grande que chez les non-tuberculeux. On peut donc affirmer que, par la présente invention on a élaboré un test de lyse immune de liposomes sansibilisés à la PPD capable de mettre en évidence des anticorps antiseracobactériens dans le serum de patients tuberculeux. On peut noter également la rapidité de ce test (30 minutes).

Ce test permet de diagnostiquer toutes les maladies infectieuses par mise en évidence d'anticorps sériques au moyen de liposomes porteurs d'antigènes du microorganisme infectant.

### 5. Test d'immunoréactivité des liposomes in vivo.

On sait que les liposomes sensibilisés par des antigènes sont susceptibles de jouer un rôle dans les mécanismes cellulaires de l'immunité in vivo, soit en imitant l'agent infectieux (bicouche de phospholipide porteuse de molécules immunogènes de cet agent) et en étant captés par les macrophages locaux de l'hête infecté, soit en imitant ces macrophages qui auraient "processé" l'agent infectieux et qui auraient exposé certaines de ses molécules antigéniques à leur surface. Dans les deux cas, les molécules antigéniques par lesquelles le liposome a été sensibilisé sont présentées aux lymphocytes, reconnues par des récepteurs et les mécanismes d'immunité cellulaire et humorale peuvent être stimulés. A ce titre, le liposome sensibilisé peut constituer le véhicule d'un nouveau type de vaccination, car il représente un modèle quasi naturel de présentation de molécules antigéniques au système immunitaire.

Si cette hypothèse est correcte, le liposome-PPD doit pouvoir induire une réaction positive d'hypersensibilité retardée (intradermoréaction positive) chez des animaux préalablement sensibilisés par le B.C.G.

On a donc injecté dans le derme des liposomes sensibilisés à la PPD (Egg Pc sensibilisés par encapsulation directe de la PPD ou par préformation suivie de mise en contact; Egg Pc/Ch, 4 : 3, sensibilisés selon les deux mêmes méthodes) à des cobayes immunisés. Les 4 types de liposomes provoquent une intradermoréaction dont l'intensité et l'évolution sont différentes de cettes de la PPD non encapsulée (Voir à ce propos, le projet d'article "Immunological activity of tuberculin sensitized liposomes", notamment p. 7 et Fig. 1 et 2, pour les liposomes Egg Pc). Par contre, les liposomes non porteurs d'antigènes de PPD ne suscitent pas d'intradermoréaction positive chez les mêmes animaux (Idem, Fig. 1).

On peut conclure que les liposomes-PPD déposés dans le derme sont phagocytés par les macrophages et que les antigènes dont ces liposomes étaient porteurs sont ensuite présentés à et appellent les lymphocytes sensibilisés, suscitant ainsi l'apparition de l'érythème et de l'induration caractéristiques de l'intradermoréaction positive.

Cette conclusion permet donc l'utilisation du liposome comme véhicule de vaccination.

### 6. Apparition d'une immunité cellulaire chez le cobaye sous l'effet de liposomes-PPD

Des cobayes non infectés et non sensibilisés par la mycobactérie tuberculeuse (Intradermoréaction négative à la PPD) ont reçu 3 injections bihebdomadaires de liposomes-PPD, soit sous la peau, soit dans la cavité péritonéale (liposomes Egg Pc en Egg Pc/Ch, 4 : 3, formés dans les deux conditions décrites plus haut, voir 1). Deux semaines après la dernière injection (ou 6 semaines après la première), une intradermoréaction a été pratiquée, avec de la PPD seule, avec des liposomes-PPD, ou avec des liposomes. Tout comme des cobayes qui auraient préalablement été sensibilisés par la mycobactérie (vaccination au B.C.G., p. ex.), ces animaux présentent une intradermoréaction positive à la PPD ou aux liposomes-PPD, mais pas aux liposomes non porteurs de PPD. Ces expériences indiquent que les liposomes sensibilisés à la PPD induisent une défense de type immunité cellulaire chez les cobayes. Rappelons que la PPD seule est incapable de susciter la moindre apparition d'une immunité chez un animal (ou un homme) qui n'a pas été préalablement mis en contact avec la mycobactérie.

On peut donc estimer que le liposome-PPD constitue un agent de vaccination plus sûr que l'actuel vaccin B.C.G. où des mycobactéries atténuées sont administrées, car le liposome-PPD ne comporte que des lipides (Egg Pc,Ch) et des protéines extraites de Mycobactéries, à l'exclusion de toute mycobactérie et de son matériel génétique.

La technique peut s'appliquer autant à la "Old Tuberculin", qui compte les protéines de la bactérie ainsi que ses polysaccharides et glycolipides de surface dont le caractère d'immunogénicité générale et aspécifique est bien connu, qu'à des molécules antigéniques spécifiques de Mycobacterium tuberculos Un liposome sensibilisé à la "Old Tuberculin" imite donc au mieux la bicouche phospholipidique de la membrane mycobactérienne portant les protéines, les polysaccharides et les lipides de cet agent infectieux intracellulaire. En suscitant une réaction portant sur l'immunité cellulaire, on dispose ainsi d'un vaccin afficace contre le parasite intracellulaire qu'est Mycobacterium tuberculosis.

D'autre part, on sait que le Mycobacterium leprae (lèpre) est très semblable à M. tuberculosis. Il existe une lépromine, semblable dans sa cmposition à la Old Tuberculin. La formation de liposomes-lépromine convient comme moyen d'immunisation contre la lèpre, maladie pour laquelle il n'existe, à ce jour, aucun vaccin efficace.

La technique de l'invention trouve également son application comme procédé de vaccination pour les affections résultant des parasites intracellulaires repris dans le Tableau I annexé :

I. Mycoses

1. Cryptococcus neoformans :                     Cryptococcose

   - germe opportuniste

   - porte d'entrée pulmonaire chez l'immunodéprimé avec généralisation dans 90 %
     des cas-méningite grave

2. Candida albicans (essentiellement) :          Candidose

   - germe surtout opportuniste : patients diabétiques, traités aux corticoïdes,
     par des antibiotiques à large spectre

   - atteinte d'abord cutanée ou muqueuse (peau, bouche, oesophage, vagin, trac-
     tus respiratoire); dissémination possible au niveau systémique (localisations
     préférentielles : reins, yeux, méninges, peau et myocarde)

3. Histoplasma capsulatum :                      Histoplasmose (USA)

   - atteinte pulmonaire avec signes généraux importants

   - dissémination systémique possible

4. Coccidioïdes immitis :                        Coccidioidomycose

   - atteinte pulmonaire (kyste - abcès...)

II. Bactéries

5. Salmonella :                                  Salmonellose

   - gastroentérite, atteintes secondaires : ostéomyélite, anévrysme

6. Listeria monocytogenes :                      Listériose

   - septicémie - méningite, affection grave chez les immunodéprimés

7. Brucella (suis, melitensis, abortus) :        Brucellose

   - maladie granulomateuse chronique généralisée (rate, os...)

8. Francisella tularensis :                      Tularémie

   - porte d'entrée cutanée ou pulmonaire, abcédation locale, adénopathies
     régionales

III. Chlamydiae

9. Chlamydiae (Bedsoniae)                        Psittacose

   - Pneumonie (rarement hépatite , encéphalite)

IV. Rickettsiae

10. Rickettsiae ( prowazekii...)                 Rickettsiose

    - température, céphalées, rash

11. Coxiella burnetii                            Fièvre Q

    - température, fièvre, pneumonie, hépatite

V. Mycobacterioses

12. Mycobacteria : - tuberculosis, bovis : tuberculose

                   - leprae :           lèpre

Dans tous les cas, il convient de noter que le même liposome sensibilisé par les antigènes d'un parasite donné peut servir tant à la vaccination qu'à la détection d'Ig (lytiques ou non lytiques) dans le serum humain.

7. Vaccin et diagnostic pour le SIDA

L'encapsulation d'un extrait de virus de SIDA (HTLV3) peut se faire au moyen d'octylglucoside. Le mode de préparation ci-après est donné à simple titre d'exemple non limitatif.

La préparation des liposomes est effectuée ici en deux temps :

a) préparation de liposomes monolamellaires par la méthode d'injection

b) fixation dans la bicouche liposomiale de l'extrait d'HTLV3 par ses molécules à composante hydrophobe après formation d'un complexe glycoprotéine - détergent et "fluidification" des liposomes.

a) Préparation des liposomes

Les lipides sont solubilisés dans l'octylglucoside (octyl-β-D-glucopyranoside) à 20°C, dans un tampon tris-HCl pH=7,2 contenant 100 mM d'octylglucoside afin d'obtenir les concentrations suivantes :

1,5 µM/ml Chol.

21 µM/ml Egg Pc.

On mélange ensuite 0,375 µ M de cholestérol et 3 m d'Egg Pc. Le tout est ensuite injecté doucement dans 3 ml de tampon phosphate (TP) pH=7,4 (NaCl=0,137 M, KCl=8,7 mM, KH$_2$PO$_4$=1,47 mM, Na$_2$HPO$_4$=8,1 mM) refroidi à 4°C, puis dialysé contre le même tampon (TP). Les liposomes sont ensuite séparés des liquides planaires par filtration sur une colonne Sephadex-G75 préalablement équilibrée avec le tampon (TP).

b) Fixation de l'extrait d'HTLV34 sur les liposomes

L'HTLV3 a été extrait par l'octylglucoside (concentration finale 7,5 mM). Les liposomes sont fluidifiés par l'octylglucoside 1 mM. Les deux solutions sont ensuite mélangées dans des proportions de 500 µg d'extrait de HTLV3/µM de lipides. Cela signifie que 0,375 µ M de lipides sont ajoutés à 137 µ g d'extrait d'HTLVS.

Le complexe extrait-détergent et les liposomes fluidifiés sont dialysés, d'abord contre un gradient de concentration décroissante en octylglucoside (de 7,5 à 0 mM) pour former des liposomes porteurs de molécules d'HTLV3, puis contre le tampon (TP) contenant 1 mM de NaN$_3$.

Les liposomes ainsi obtenus, sensibilisés à des molécules extraites du virus du SIDA, ont été injectés I.V. à des souris. Après 3 injections de cette prépration, effectuées à 3 semaines d'intervalle, la présence d'anticorps anti-HTLV3 a été mise en évidence dans leur sérum.

On peut en conclure que cette technique constitue un moyen de vaccination anti-SIDA et de diagnostic de cette maladie, tout comme dans le das de la tuberculose.

**Revendications**

1. Agents convenant comme moyens de diagnostic et comme agents d'immunisation de parasites intracellulaires caractérisés en ce qu'il sont constitués par des liposomes sensibilisés à des molécules antigéniques spécifiques de parasites intracellulaires.

2. Agents selon la revendication 1 caractérisés en ce que les liposomes sont du type dit "multilamellaires" avec ou sans cholestérol.

3. Agents selon la revendication 2 caractérisés en ce que les liposomes sont des liposomes multilamellaires avec cholestérol présentant des rapports Egg Pc/Ch compris entre 4:1 et 4:4.

4. Agents selon la revendication 3 caractérisés en ce que les rapports Egg Pc/Ch sont égaux à 4:4 ou proches de cette valeur.

5. Procédé pour la production de liposomes sensibilisés selon l'une quelconque des revendications 1 à 4 caractérisé en ce qu'on procède à une encapsulation directe de la molécule antigénique.

6. Procédé pour la production de liposomes sensibilisés selon l'une quelconque des revendications 1 à 4 caractérisé en ce qu'on met en contact le liposome préformé avec la molécule antigénique.

7. Procédé selon l'une quelconque des revendications 5 ou 6 caractérisé en ce que la molécule antigénique est constituée par la tuberculine, la Old tuberculin, la lépromine ou le HTLV3.

8. Application des agents selon l'une quelconque des revendications 1 à 4 comme moyen de diagnostic des infections s'accompagnant de formation d'immunoglobulines spécifiques dirigées contre les agents viraux, bactériens et mycotiques, et comme moyens d'immunisation (vaccination) pour les pathogènes intracellulaires entraînant l'apparition d'une immunité cellulaire.

Office européen
des brevets

RAPPORT DE RECHERCHE EUROPEENNE

Numero de la demande

EP  88 87 0002

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | EP-A-0 203 676  (THE WISTAR INSTITUTE OF ANATOMY AND BIOLOGY) <br> * Page 1, lignes 1-11; page 3, ligne 25 - page 6, ligne 14; revendications 1,5 * <br> --- | 1-8 | A 61 K  39/00 <br> A 61 K  39/002 <br> A 61 K  39/02 <br> A 61 K  39/12 <br> G 01 N  33/569 <br> C 07 K  17/00 |
| X | CHEMICAL ABSTRACTS, vol. 103, no. 21, 25 novembre 1985, page 556, résumé no. 176865v, Columbus, Ohio, US; M. PIMM et al.: "Liposomal encapsulation augments delayed hypersensitivity reactions to tuberculin proteins", & J. MED. MICROBIOL. 1984, 18(3), 429-32 <br> * Résumé * <br> --- | 1-8 | |
| X | EP-A-0 097 407  (RIJKSUNIVERSITEIT UTRECHT) <br> * Page 2, lignes 9-15; revendications 1-3,6,9 * <br> --- | 1-4,6,8 | |
| X | EP-A-0 047 480  (INSTITUT ARMAND FRAPPIER) <br> * Page 1, lignes 4-10; page 4, lignes 22-27,29-33; revendications 1,4,9 * <br> --- | 1-4,6,8 | DOMAINES TECHNIQUES RECHERCHES (Int. Cl.4) <br><br> A 61 K |
| X | EP-A-0 017 557  (MERCK & CO, INC.) <br> * Page 1, lignes 4-14; page 2, ligne 30 - page 3, ligne 32; revendications 1-6 * <br> --- | 1-4,6,8 | |
| X | US-A-4 261 975  (W.W. FULLERTON et al.) <br> * Colonne 1, lignes 17-40; revendications 1-6 * <br> --- | 1,2,5,8 | |
| X | US-A-4 117 113  (A.C. ALLISON et al.) <br> * Colonne 1, lignes 40-63; colonne 2, lignes 41-52; revendications 1,3 * <br> --- -/- | 1,2,5,8 | |

Le présent rapport a été établi pour toutes les revendications

| Lieu de la recherche | Date d'achèvement de la recherche | Examinateur |
|---|---|---|
| LA HAYE | 25-04-1988 | CHARLES D.J.P.I.G. |

Office européen
des brevets

RAPPORT DE RECHERCHE EUROPEENNE

Numero de la demande

EP 88 87 0002

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | BIOLOGICAL ABSTRACTS, vol. 82, no. 11, 1986, résumé no. 105543, Biological Abstracts, INC., Philadelphie, P.A., US; C.R. ALVING et al.: "Effectiveness of liposomes as potential carriers of vaccines: applications to cholera toxin and human malaria sporozoite antigen", & VACCINE 4(3): 166-172 * Résumé * --- | 1-6,8 | |
| X | BIOLOGICAL ABSTRACTS, vol. 74, no. 10, 1982, résumé no. 68612, Biological Abstracts, INC., Philadelphie, P.A., US; P.T. NAYLOR et al.: "In vivo induction of anti-herpes simplex virus immune response by type 1 antigens and lipid A incorporated into liposomes", & INFECT. IMMUN. 36(3): 1209-1216 * Résumé * --- | 1-5,8 | |
| X,P | US-A-4 663 161 (R.J. MANNINO et al.) * Colonne 1, ligne 65 - colonne 2, ligne 27; colonne 3, lignes 4-28; revendications 1-7 * ----- | 1-8 | DOMAINES TECHNIQUES RECHERCHES (Int. Cl.4) |

Le présent rapport a été établi pour toutes les revendications

| Lieu de la recherche | Date d'achèvement de la recherche | Examinateur |
|---|---|---|
| LA HAYE | 25-04-1988 | CHARLES D.J.P.I.G. |

EPO FORM 1503 03.82 (P0402)